Europäisches Patentamt

**European Patent Office**

**Office européen des brevets**

(19)

(11) Publication number: **0 223 023**
**A1**

(12) # EUROPEAN PATENT APPLICATION

(21) Application number: **86113491.4**

(22) Date of filing: **01.10.86**

(51) Int. Cl.⁴: **A 61 M 25/00**

(30) Priority: **02.10.85 IT 2329885 U**

(43) Date of publication of application: **27.05.87**
**Bulletin 87/22**

(84) Designated Contracting States: **AT BE CH DE ES FR GB GR IT LI LU NL SE**

(71) Applicant: **Pierrel Hospital SpA, S.S. Stelvio, I-23035 Sondalo (IT)**

(72) Inventor: **Serafini, Giampaolo, Via Chiozzo 48/A, I-27100 Pavia (IT)**

(74) Representative: **Gudel, Diether, Dr. et al, Patentanwälte Dr. V. Schmied-Kowarzik Dipl.-Ing. G. Dannenberg Dr. P. Weinhold Dr. D. Gudel Dipl.-Ing. S. Schubert Dr. P. Barz Grosse Eschenheimer Strasse 39, D-6000 Frankfurt am Main 1 (DE)**

(54) **Central venous catheter.**

(57)    By taking advantage of the electric conductivity of a liquid filling a venous catheter which is inserted in the usual way up to the right auricle of the hearth, this liquid being into contact with metal components conducting the electric signals of miocardial depolarization and repolarization to an outer electrocardiographic monitoring apparatus, it is possible to control the positioning of the central venous catheter during the inserting and precision positioning manoeuver.

EP 0 223 023 A1

The present invention relates to a central venous catheter and more particularly to a central venous catheter with possibility of positioning onto the guide of the electrocardiograph. The central venous catheterism consists in bringing within the central venous system (lower hollow vein, upper hollow vein or right hearth auricle) a catheter inserted, by percutaneous route by means of a needle, in a peripheral vein.

The access venous routes used are multiple. It is possibile to puncture a peripheral surface venous vessel (basilica vein, cephalic vein, saphenous vein) or a deep venous vessel (femoral, outer or inner jugular, axillary or subclavian vein).

Aubaniac in 1952 and Keer-Szanto in 1956 were the first using the subclavian vein, by subclavicular route, as an infusion route, putting the basis for the catheterization of the right auricle. Since thirty years the method of Aubaniac is still that mostly used owing to its minor risk of complications and to the greater nursing easiness.

Whatever is the route used, the central venous catheterism today represents an essential instrument in order to mainly achieve four objectives:

1) monitoring of the central venous pressure (CVP)

2) restoring the volemic and electrolytic balance under urgency conditions

3) carrying out the total parenteral nutrition (TPN)

4) infusing drugs which are phlebotoxic if injected in vessels of narrow cross-section.

The essential feature, upon the inserting manoeuver is carried out, is that of the control of the exact positioning of the point of the catheter which, if placed too much distally (within the right ventricle) may give place to even serious arithmic troubles; if it is placed too much proximally does not attain the purpose sought for by this technique.

A further problem arises if the point of the catheter is accidentally positioned in a vessel different from the central ones (counterside subclavian, inner or outer jugular vein).

Several authors report that the percentages of erroneous positioning vary between 2% and 20% of cases.

The only method to date used and capable of evidencing a wrong positioning of the catheter consists in the radiological control upon the manoeuver is carried out.

The economical charge resulting from such a type of control is self-evident use of apparatus and personal in the radiological division, repeating the manoeuver in the case of wrong positioning and possible further radiological control. Besides the economical point of view, such a control does also and mainly heavily weigh in terms of radiations administered to the patients. Such a problem is even more serious in the case of patients undergoing anti-neoplasic and immunosuppressing therapies.

The purpose of the present invention is that of providing a non invading technique, by which it is possible to evidence the arrival of the point of the catheter in the right auricle of the hearth (such a condition being necessary to ensure the good positioning) during the carrying out of the manoeuver, thus avoiding all the drawbacks to date arising in the positioning of central venous catheters.

Such a purpose is achieved with the central venous catheter according to the present invention, characterized by comprising a tubular member, preferably of biocompatible plastic material, open at the end to be positioned within the right auricle of the hearth and having at the opposite end, to be into contact with the outer surface of the skin, first means for the electric connection to an electrocardiographic control apparatus and second means for the introduction into said tubular member of an electroconducting liquid, preferably physiological solution. According to the preferred embodiment of the present invention the venous catheter comprises a tubular member of biocompatible plastic material to be filled with physiological solution having at the proximal end a hollow metal sleeve coupled thereto, the latter being joined at the other end to a plastic cone, adapted to receive a syringl or like means for the filling in of physiological solution, an electrically conductive element being provided on the cone and being electrically connected on one side to the said sleeve and on the other side to the electrocardiographic control apparatus.

As it shall appear more clearly from the following detailed description, the central venous catheter according to the present invention permits a non invasive technique to be used, based on the principle of the endocavitary electrocardiography. As it has been confirmed by the practicing, of the invention the venous catheter, upon it is filled with physiological solution, does act as a conducting wire, which is separated and isolated from the blood stream by the same plastic material forming it (polyethylene, polyethylene therephtalate, polyvinyl chloride).

The open point at the distal end acts as an electrode detecting the variations of electric potential occurring in sequence during the phases of miocardial depolarization and repolarization.

Such a point thus constitutes an electrode which, if inserted up to the right auricle, behaves as an intracavitary electrode; the electric phenomena are trasferred, thanks to the column of physiological solution, to the proximal end of the catheter, which comes out of the skin, and therefrom through a standard electrocardiographic cable to a monitoring display screen.

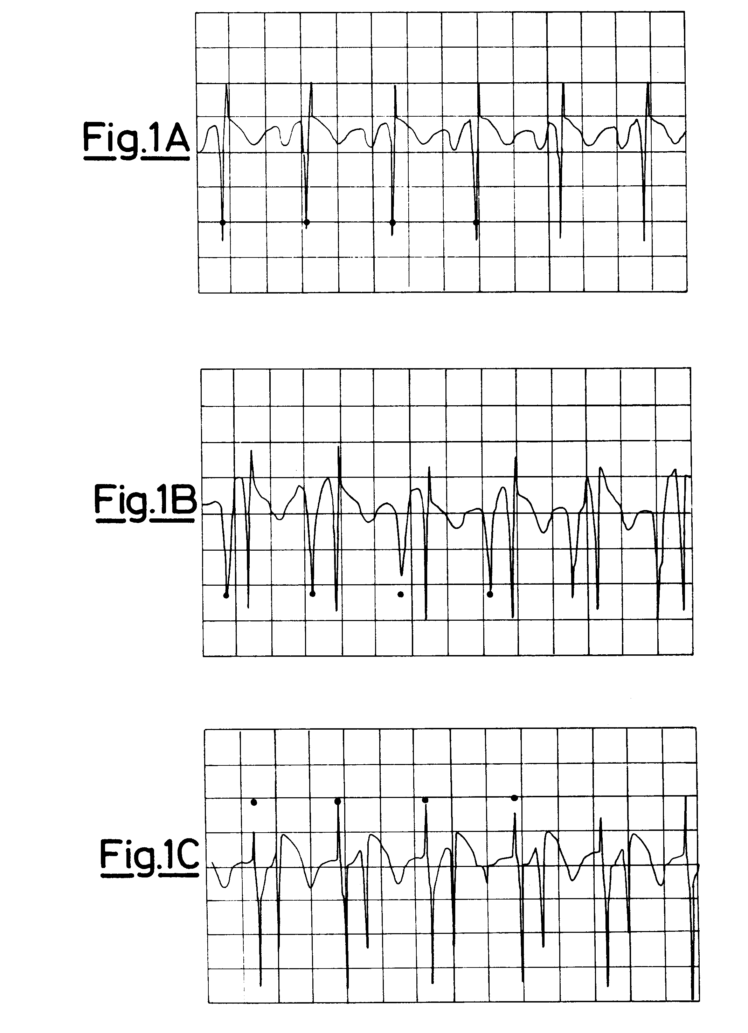

In the figures 1A, 1B and 1C, the electrocardiographic variations which can be detected and recorded are shown in sequence.

In fig. 1A the point of the catheter is positioned in the upper hollow vein and no significant feature is shown by the plotting. In fig. 1B the point arrives within the right auricle at the level of the sino-auricle knot: the P wave shows an abrupt negative deflexion (intrinsecoidal deflexion of the P-wave). In fig. 1C the advancement of the point to the mean area of the right auricle is indicated by the appearance of a progressively positive deflexion.

Referring now to the drawings, which specifically illustrate, but for purely exemplifying purpose, the venous catheter of the invention:

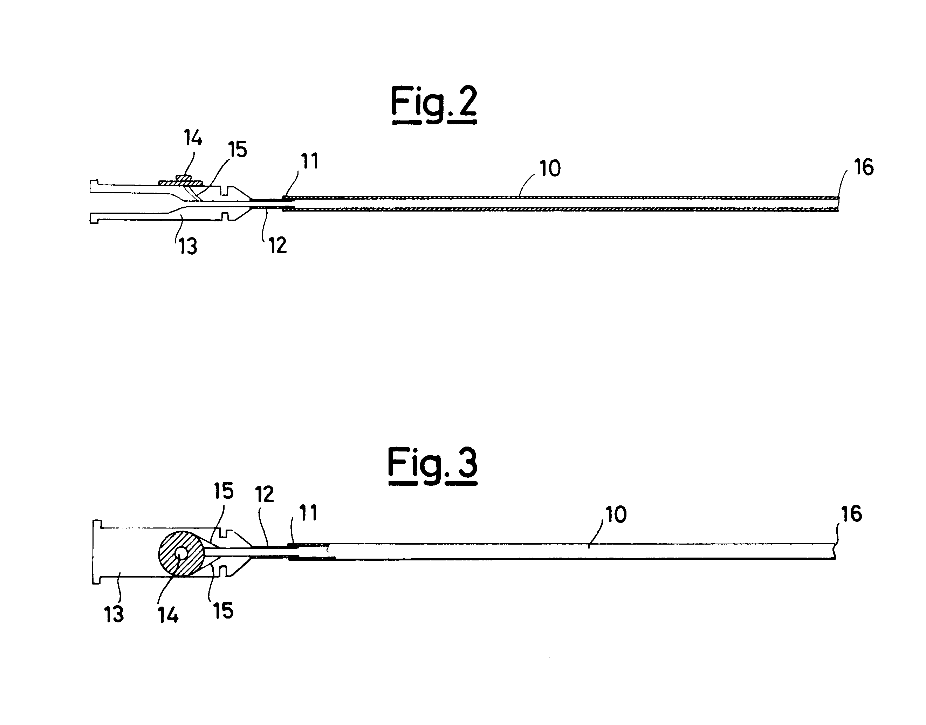

fig. 2 is a longitudinal axial section view of the catheter according to a preferred embodiment, and fig. 3 is a plan view from above of the catheter of fig. 2.

In the drawings the central venous catheter comprises a tubular member 10 of electrically isolating and biocompatible material, preferably a plastic material selected among polyethylene therephtalate, polyvinyl chloride and polyethylene. At the proximal end 11 a sleeve 12 of electrically conductive material is coupled,it being preferably metal. The opposite end 12 of the sleeve 12 is anchored within a cone of plastic material 13.

Into this cone there is inserted (during the positioning manoeuver) either a syringe or the flow set of a phleboclysis device. The surface of the cone 13 is shaped so as to be able to receive a metal button 14 like those of the adhesive cutaneous electrodes. Such a button is in contact through two thin wires 15 passing inside the cone 13, with the sleeve 12. In this way an electric continous path is set up, which starting from the point 16 of the catheter, extends along its lenght up to the metal sleeve 12, passes through the thin wires 15 ending at the metal button 14.

To this metal button the common cable of a cardiac monitor is joined and the electric signal can be in this way brought from the point of the catheter to an electrocardiographic apparatus.

The structural features of the catheter of the present invention are unique since, with a minimum encumbrance and without other instrumentation, an electric signal can be transmitted from within the cardiac cavities to the outer surface of the cone of the catheter and therefrom to the monitor.

In order to give a general indication of the preferred embodiment of the central venous catheter of the present invention:

-) the true catheter, having a lenght of between 20 and 30 cm and a diameter of between 0.7 and 1.5 mm, is of the above mentioned plastic material.

-) At the proximal end of the catheter, placed outwardly of the skin, the

0223023

hollow metal sleeve is coupled, having a lenght of about 2 cm.

-) In turn the sleeve is inserted into the said cone of plastic material which, at the opposite end, receives a syringe or the flow set of a phleboclysis device.

-) Onto the surface of the plastic cone a metal button is seated, having round shape and being like the standard cutaneous electrodes.

-) Such a button is in contact, through at least one conducting wire, with the sleeve coupled to the catheter.

In this manner an electric path is created from the column of physiological solution to the metal sleeve, to the conducting wire up to the button seated onto the cone surface.

To this button the tweezers of the electrocardiographic cable are coupled, whereby the signal is transmitted to the electrocardiographic screen. Upon the positioning manoeuver is completed, the plastic cone with the metal button attached thereto is removed for reasons of electric safety.

The catheter of the invention is distinguished in that a continuous monitoring of the advancing of the catheter towards the hearth and thus controlling, at any time, of the precise positioning is permitted, whereby a possible wrong positioning can be immediately detected, without any radiological control.

Obviously conceptually and structurally equivalent variations are possible and foreseable in the catheter of the invention; for instance the sleeve can be of plastic material, internally bored and metallized, the axial bore being essential in order to ensure the perfusion condition which must exist since the first introduction of the catheter.

CLAIMS

1. Central venous catheter, characterized by comprising a tubular member, open at the end to be positioned within the right auricle of the hearth and having at the opposite end, to be into contact with the outer surface of the skin, first means for the electric connection to an electrocardiographic control apparatus and second means for the introduction into said tubular member of an electroconducting liquid.

2. Central venous catheter, according to claim 1, characterized in that said isolating material is biocompatible plastic material.

3. Central venous catheter according to claim 2, characterized in that said plastic material is selected among polyethylene therephtalate, polyvinyl chloride and polyethylene.

4. Central venous catheter, according to claim 1, characterized in that said first means comprise a metal sleeve having one end inserted into said tubular member and the opposite end electrically connected to the sensing electrode of an electrocardiographic monitoring apparatus.

5. Central venous catheter according to claim 4, characterized in that said electrical connection comprises a cone of plastic material in which said opposite end of said metal sleeve is inserted, said cone protruding outwardly of the skin, of the patient a metal button being seated onto said cone and connected, internally of said cone, with said metal sleeve by at least one electrically conductive wire, said metal button being in turn connected to the common cable of a cardiac monitor.

6. Central venous catheter, according to claim 5, characterized in that said cone of plastic material is shaped so as to permit the introduction of a syringe for filling in said electrically conductive liquid and/or other perfusing means.

7. Central venous catheter according to claim 1, characterized in that said electric conductive liquid is physiological solution.

0223023

Fig.1A

Fig.1B

Fig.1C

## Fig.2

14  15  11  10  16

13  12

## Fig.3

15  12  11  10  16

13  14  15

0223023

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 134 845 (SPAA) <br> * page 1, lines 1-12; abstract * | 1,2,4 | A 61 M 25/00 |
| A | US-A-3 807 391 (BOLDUC) <br> * claims 1-5; figure 2 * | 1 | |
| A | FR-A-2 272 690 (GRAUSZ et al.) <br> * claims 1, 2, 4-6 * | 1-4 | |

TECHNICAL FIELDS SEARCHED (Int. Cl.4)

A 61 B 5/04
A 61 B 17/39
A 61 M 25/00
A 61 N 1/05

The present search report has been drawn up for all claims

| Place of search | Date of completion of the search | Examiner |
|---|---|---|
| BERLIN | 19-01-1987 | PAPA E.R. |